# EUROPEAN PATENT APPLICATION

(11) **EP 2 316 332 A1**
(43) Date of publication of application: **04.05.2011**
(21) Application number: 09174580.2
(22) Date of filing: 30.10.2009
(51) Int. Cl.: A61B 5/00

(54) **Sensor for measuring amount of substance in blood and method of making the sensor**

(71) Applicant: General Electric Company, Schenectady, NY 12345 (US)
(72) Inventor: Kåll, Magnus, 02770 Espoo (FI)
(74) Representative: Valkeiskangas, Tapio Lassi Paavali

(57) **Abstract**

The invention relates to sensor and method for measuring amount of substance in blood. The sensor comprises a base element (9) having a surface, and at least one light emitting diode (10) emitting at pre-determined wavelength, and a photodetector (11) which is sensitive to the pre-determined wavelength. The at least one light emitting diode comprises at least one emissive layer having been printed onto the surface.

## Description

### BACKGROUND OF THE INVENTION

The disclosure relates to a sensor for measuring amount of substance in tissue the sensor comprising:
a base element having a surface, and
at least one light emitting diode emitting at pre-determined wavelength and a photodetector which is sensitive to the pre-determined wavelength.

Pulse oximetry is a well-established technique for measuring oxygen saturation (SpO₂) in arterial blood, SpO₂ is an important parameter that relates to the adequacy of oxygen supply to peripheral tissues and organs. Pulse oximeters provide instantaneous in-vivo measurements of arterial oxygenation, and thereby an early warning of arterial hypoxemia, for example. Pulse oximeters also display a photoplethysmographic (PPG) pulse waveform, which can be related to tissue blood volume and blood flow, i.e. the blood circulation, at the site of the measurement, typically in finger or ear.

Since the measurement is normally made from an anatomical extremity, such as a finger tip, pulse oximeters typically comprise a separate sensor attachable to a subject and the actual pulse oximeter device to which the sensor is connected through a cable. Standard pulse oximeters use two wavelengths to measure the ratio of oxyhemoglobin to total functional hemoglobin, indicated as an SpO₂ value. The sensor of a standard pulse oximeter therefore comprises two emitter elements, each emitting radiation at a specific wavelength, and a photodetector common to the emitter elements. Although standard pulse oximeters require only two wavelengths, multiwavelength pulse oximeters provided with more than two wavelengths are becoming more and more common, since they provide higher performance and wider applicability. For example, levels of other significant hemoglobin species, such as carboxy-hemoglobin and methemoglobin and total hemoglobin, may be estimated if the number of wavelengths used in the pulse oximeter is increased. The sensor of a multiwavelength pulse oximeter therefore comprises more than two, typically 6 to 12, emitter elements, and a broad spectral band photodetector common to all emitter elements.

A pulse oximeter normally comprises a bedside monitoring unit and a probe or sensor unit attachable to a subject, typically to a finger or ear lobe of the subject. The sensor unit is normally connected to the monitoring unit through a cable. The monitoring unit may be conceived to comprise three basic elements: a computerized control and processing unit, a memory for the control and processing unit, and a display for displaying information to a user of the pulse oximeter.

The sensor unit normally includes light sources for sending optical signals through the tissue and a photodetector for receiving the signals transmitted through or reflected from the tissue. On the basis of the transmitted and received signals, light absorption by the tissue may be determined. During each cardiac cycle, light absorption by the tissue varies cyclically. During the diastolic phase, absorption is caused by venous blood, non-pulsating arterial blood, cells and fluids in tissue, bone, and pigments, whereas during the systolic phase there is an increase in absorption, which is caused by the inflow of arterial blood into the tissue part on which the sensor is attached. Pulse oximeters focus the measurement on this pulsating arterial blood portion by determining the difference between the peak absorption during the systolic phase and the background absorption during the diastolic phase. Pulse oximetry is thus based on the assumption that the pulsating component of the absorption is due to arterial blood only.

In order to distinguish between two species of hemoglobin, oxyhemoglobin (HbO₂) and deoxyhemoglobin (RHb), absorption must be measured at two different wavelengths, i.e. the sensor of a traditional pulse oximeter includes two different light emitting diodes (LEDs) or lasers. The wavelength values widely used are 660 nm (red) and 940 nm (infrared), as described above since the said two species of hemoglobin have substantially different absorption at these wavelengths. Each LED is illuminated in turn at a frequency which is typically several hundred Hz. If the concentrations of more than said two hemoglobin species are to be evaluated, more than two wavelengths are needed. Such a pulse oximeter is here termed a multiwavelength pulse oximeter.

The light propagated through or reflected from the tissue is received by a photodetector, which converts the optical signal received at each wavelength into an electrical signal pulse train and feeds it to an input amplifier. The amplified signal is then supplied to the control and processing unit, which converts the signals into digitized format for each wavelength channel. The digitized signal data is then utilized by an SpO₂ algorithm. The control and processing unit executes the algorithm and drives the display to present the results on the screen thereof. With each LED being illuminated at the above-mentioned high rate as compared to the pulse rate of the subject, the control and processing unit obtains a high number of samples at each wavelength for each cardiac cycle of the subject. The time windows corresponding to a particular wavelength are often referred to as a wavelength channel.

Photoplethysmography (PPG) is a non-invasive optical technique that measures variations in skin blood volume and perfusion. The (PPG) signal contains components that are synchronous with respiratory and cardiac rhythms. A photoplethysmograph can be measured using a single wavelength, typically in the infrared range. An example of a single-wavelength photoplethysmograph is described in WO/2007/122375.

In the prior art semiconductive light emitting diodes (LED's) have been traditionally used as light sources for SpO2 sensors. The LED's used in Sp02 sensors today are manufactured on a GaAs, GaP or GaAsP wafer and diced into small chips that are mounted on a substrate. The typical manufacturing process involves bonding the chip to a substrate using a conductive adhesive, curing the adhesive in an oven, bonding a gold wire from the top of the chip to a contact pad on the substrate and finally encapsulating the whole assembly in a clear encapsulant. To be able to utilize this assembly in an Sp02 sensor, it must further be connected to electrical conductors connecting the LED electrodes to an Sp02 monitor. This is typically done using soldering or wire welding. This whole process is time consuming and requires a number of automated assembly machines to enable large scale production. An example of such a sensor design is described in US Patent 6 745 061.

A plethysmography sensor consists of an infrared emitter and a detector that is sensitive to the light emitted by the emitter. There are two modes of photoplethysmography, transmission mode and reflection mode. In transmission mode the light source is on one side of the tissue and the photodetector is placed on the other side, opposite the light source. In reflection mode the light source and photodetector are placed side-by-side. Light entering the tissue is reflected and a proportion of this is detected at the photodetector. The structure of a single-wavelength plethysmography sensor is essentially the same as for two wavelength sensor, except there is only one emitter.

### BRIEF DESCRIPTION OF THE INVENTION

The above-mentioned shortcomings, disadvantages and problems are addressed herein which will be understood by reading and understanding the following specification.

In an embodiment, a sensor for measuring amount of substance in blood comprises a base element having a surface, and further at least one light emitting diode emitting at pre-determined wavelength and a photodetector, which is sensitive to the pre-determined wavelength. The at least one light emitting diode comprises at least one emissive layer. The emissive layer has been printed onto the surface.

In another embodiment, method of making a sensor for measuring amount of substance in blood comprises the steps of providing a base element having a surface, and forming a structure having layers by printing the layers onto the surface, the layers comprising at least one emissive layer having a pre-determined wavelength, and anode and cathode layers.

The embodiments described above utilize organic light emitting diodes (OLED's) to generate the light needed to measure the amount of substance in blood. OLED technology can also be used to create the photodetector used to detect the light emitted by the OLED's and attenuated by tissue. OLED's are currently commercially used in dot matrix displays and are gaining ground in general lighting applications. In an OLED, the emissive layer of the light emitting diode is an organic compound (i.e. contains carbon). An example of a display embodiment utilizing OLEDs is described in US Patent 7 321 348.

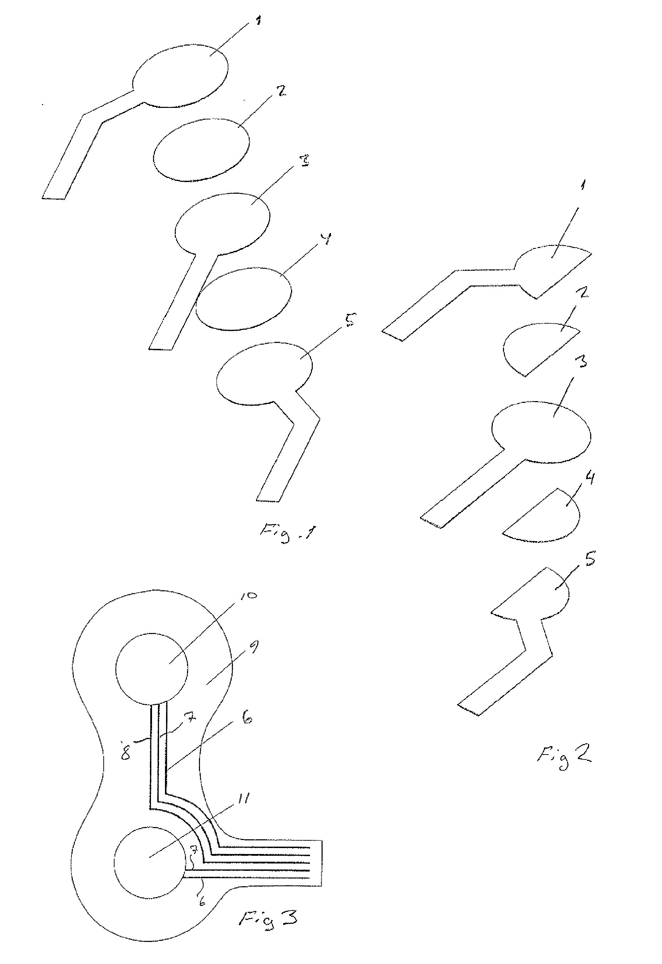

The benefit of using organic LED's is that they can be printed directly onto a flexible or rigid surface which in turn can be an integral part of an Sp02 sensor. In addition, the layers in the organic LED can be made transparent enabling the printing of several OLED's on top of each other (see figure 1). For example the anode or cathode on top of the emissive layer needs to be transparent in order for the light emitted from the emissive layer to be visible. This enables virtually identical light paths for light of different wavelengths, which is advantageous when measuring amount of substance in blood. Also, OLED's require a larger surface area to obtain the same optical output power as traditional LED's, making it advantageous to print the OLED's in layers to minimize the required surface area. Printing of OLEDs is described for example in US Patent 6 982 179.

### BRIEF DESCRIPTION OF THE DRAWINGS

Figure 1 shows a first basic principle of the placement of the layers in the sensor of one embodiment,
Figure 2 shows a second basic principle of the placement of the layers in the sensor of another embodiment,
Figure 3 shows a further embodiment of sensor made according to the principles of Figure 1,
Figures 4-8 show a basic steps how the sensors made can be made onto a continuous sheet,
Figures 9 and 10 show principally how the sensor of the invention can be wrapped around the application site, in this case around a finger,
Figure 11 shows principally a structure using a lens with sensors of the embodiments shown in previous figures, and
Figures 12-14 show some examples of for terminating conductor traces on the sheet to a cable/connector.

### DETAILED DESCRIPTION OF THE INVENTION

As discussed above a sensor for measuring amount of substance in blood is obtained with the use of OLED technology. The amount of substance in blood may be for example the ratio of oxyhemoglobin to total functional hemoglobin, indicated as an Sp02 value.

Figure 1 shows a basic principle. In the embodiment of Figure 1 at least two organic light emitting diodes and alternatively also a organic photodetector have been printed onto the surface to form light emitting diodes emitting at different wavelengths and alternatively also an organic photodetector which is sensitive to the two different wavelengths. The base element may be either a rigid element or a flexible element. The base element may also be advantageously a continuous element.

If oxygen amount in blood is measured, the wavelengths of the at least two light emitting diodes, and a spectral response of the photodetector are typically within the ranges of 650 ... 670 nm and 880 ... 950 nm.

It is also advantageous to make at least one of the printed layers of the organic light emitting diodes, for example emissive layers, cathodes and anodes as transparent elements, i.e. by forming the diodes and/or detectors by using at least one transparent printed layer, because said feature enables that at least part of the layers can be printed on top of each other to obtain good light path properties. The anode or cathode on top of the emissive layer needs to be transparent in order for the light emitted from the emissive layer to be visible. It is advantageous that the layers are made transparent so that all layers can be printed on top of each other.

A lens can also be arranged on top of the organic light emitting diode/diodes and/or the organic photodetector to obtain good focusing properties. The organic light emitting diode/diodes and organic photodetector printed may also comprise printed conductive traces for connectors and/or electrical wires.

Figures show embodiments using the features discussed above.

By printing the emitter/emitters and detector(s) directly onto the sensor, the manufacturing process for an Sp02 sensor becomes much simpler. This would be especially advantageous for disposable Sp02 sensors. Figure 1 shows a schematic example of a disposable Sp02 sensor. The sensor shown in Figures 1 and 2 comprises five layers. Layer 1 is the anode for the red OLED, layer 2 is an emissive layer (red), layer 3 is a common cathode, layer 4 an emissive layer (infrared) and layer 5 is the anode for the infrared (IR) OLED. The layers described above are printed successively onto the surface of a base element 9 and on top of each other. The manufacturing steps, i.e. the printing step for manufacturing a disposable OLED Sp02 sensor would also comprise of printing conductive traces 6, 7, 8, OLED layers 1 - 5 and potential graphics on the surface of the base element 9. The conductive traces mentioned can be printed as a separate layer. This is however not the only alternative, but the conductive traces can for example be part of the electrode layer. The base element may be continuous polymer sheet, for example continuous polyester sheet. In this embodiment the at least the layers 1, 2, 3 and 4 are transparent layers. The layer 5 can be transparent or non-transparent.

In case of a photoplethysmograph, only one wavelength is required, hence the amount of layers needed is reduced, i.e. in the case of a photoplethysmograph, only layers 1 ― 3 are required anode, emissive layer and cathode.

Figure 2 shows a basic principle of a second embodiment. In the embodiment of Figure 2 the emissive layers 2 and 4 are not transparent. The emissive layers 2 and 3 have been placed side by side with respect to each other. The anode for the red OLED 1 and the anode for infrared OLED has been formed according to the emissive layers and placed on top of each other as shown in Figure 2. In this embodiment at least the layers 1 and 3 are transparent. The layer 5 can be transparent or non-transparent.

The phrase "side by side" must be understood broadly, i.e. the phrase means here that the emissive layers 2, 4 are not placed on top of each other. The emissive layers can be placed side by side so that there is no space between the layers when looking at horizontally, or the layers can be placed side by side so that there is a space between the layers. Figure 2 shows the arrangement principally.

Figure 3 shows an example of how the structures of Figures 1 and 2 could be arranged on a continuous sheet 9. Reference number 10 shows an emitter and reference number 11 detector.

The following example describes the solution on a theoretical level. As described above the printing would comprise also a conductive trace layer to provide electrical contact between the optical components and the cable/monitor connector. The second layer would consist of either the anode or cathode for the organic LED or photodiode. The third layer is the emissive layer of the OLED (the composition of this layer determines the wavelength of the emitted light). Subsequent layers are a photosensitive layer for the photodiode, anodes/cathodes for both LED's and photodiode, further stacks of emissive and anode/cathode layers if several wavelengths are used. The minimum amount of layers for an Sp02 sensor in the example described would be 7 (traces, anodes, emissive (red), photosensitive, cathodes, emissive (IR), anode).

Subsequent process steps would consist of attaching or printing an adhesive and attaching a release liner onto the sensor. If a separate connector is used, it can be attached to the sheet at this time after which the sensor is ready for singulation. After all components have been printed/assembled, each sensor is punched out from the sheet and packaged for shipment.

The structure described can also be used so that the emitter is formed in the way discussed above and the detector used is a traditional photosensitive detector. It is also possible that the detector is formed in the way discussed above and the emitter is a traditional emitter, i.e. both the emitter and the detector can be formed as described above or either the emitter alone or the detector alone is formed in the way described above. The number of layers is naturally less than described in the example above if traditional detector is used.

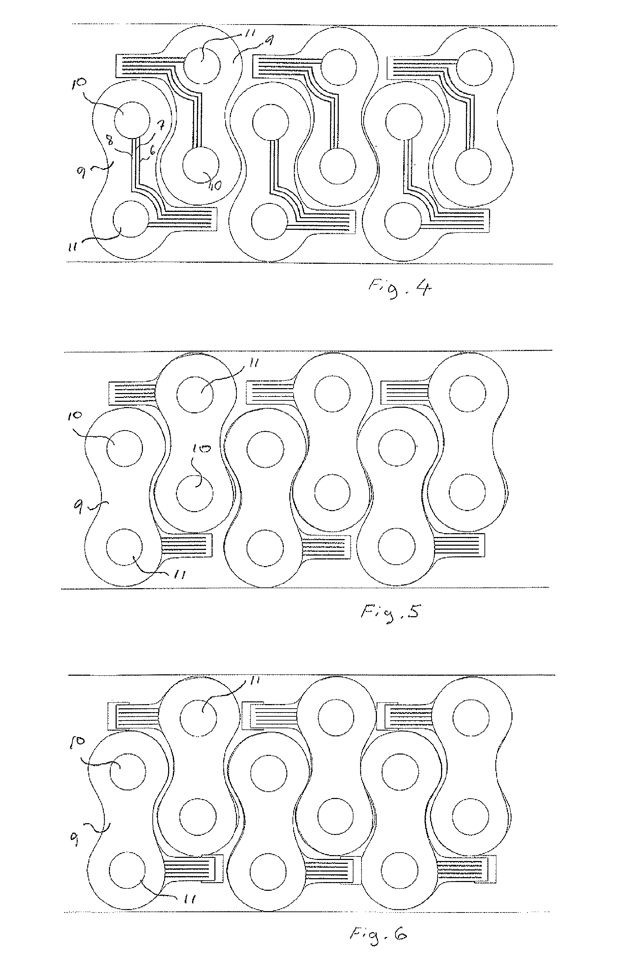

The process described above is principally described in Figures 4 -8. In this embodiment the base element is a flexible substrate rolled out from an appropriate storage, for example a roll. Figures 4 - 8 below show a section of the reel-to-reel polymer sheet where OLED Sp02 sensors are printed and processed.

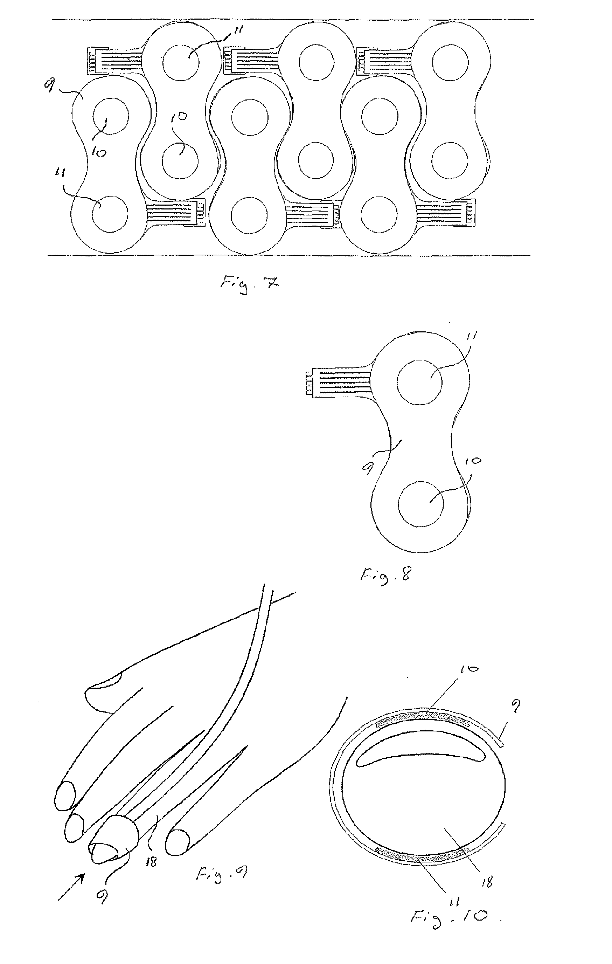

An example of the manufacturing steps for manufacturing a printed disposable OLED Sp02 sensor could be for example:
1. Print graphics, electrodes, emissive layers, insulators and conductive traces on a polymer sheet in a reel-to-reel fashion (Figure 4).
2. Print or apply adhesive layers for attaching the sheet to the skin or additional structural materials (optional) such as foam or other polymer sheets (Figure 5).
3. Partial singulation to enable wire or connector termination (Figure 6) (optional step).
4. Application of wires or contacts to the sheet for connection to patient monitor (Figure 7) (optional step).
5. Singulation of finished sensor assembly or sub-assembly (Figure 8).

Since the organic LED's can be printed onto elastic materials, the emitter can be made to elastically bend around the application site, minimizing the risk of ambient light interference. The same applies of the organic photodiode. This matter is shown in Figures 9 and 10 which show a finger 18 and the sensor wrapped around said finger.

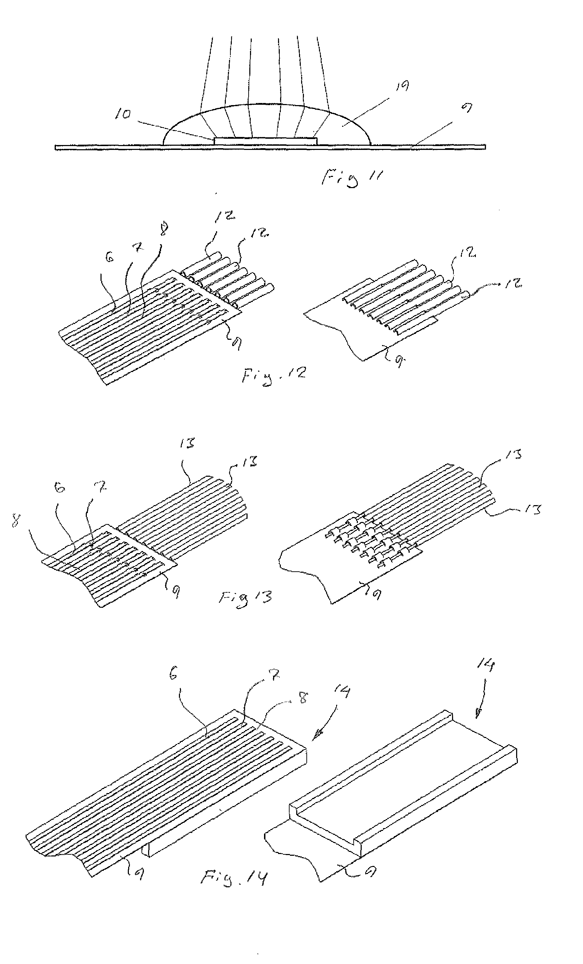

If the application requires the light to be focused in a particular way, a lens 19 can be molded on top of, or adhered to the surface of the organic LED or photodetector. This would enable focusing light from a larger surface area into a more focused beam enabling deeper tissue penetration. This matter is described in Figure 11 showing said lens 19 fastened on top of the sensor, in this embodiment emitter 10 made as described above. The term lens must be understood widely here, i.e, said term means here also for example lens assemblies made of two or more lens components etc. Lines in Figure 11 show path of the light emitted.

As the conductive electrodes are printed onto the sensor, printing could also be used to provide the electrical conductors from the sensor connector to the OLED's and photodetector. This way there would not be any need for separate connections between the emitter/detector and the sensor wiring to complete the circuit. The printed conductive traces could be terminated directly to contact pins or to wires connected to a connector. Preferably the connector could be molded directly around the substrate utilizing the printed traces as contacts. Figures 12 - 14 show different connector termination options to the base element 9, for example polymer sheet. Figures 12-14 show the embodiment in question seen from above and below. Figure 12 shows sockets or pins 12 crimped directly to the sheet 9 on top of the traces 6, 7, 8 to form an electrical connection between the socket/pin and the trace. Stamping or riveting can alternatively be used to attach the sockets or pins to the sheet. The contact pins can further be inserted into a connector frame or overmolded to form a connector. Figure 13 shows wires 13 directly crimped to the traces 6, 7, 8 on the sheet 9. This enables the wires to be terminated into traditional contact sockets or pins. The wire crimps can be covered with adhesive or tape for protection. Figure 14 shows a connector frame 14 attached directly to the sheet. In this embodiment, the traces 6, 7, 8 themselves act as electrical contact pads for the connector resulting in a low cost solution.

This written description uses examples to disclose the invention, including the best mode, and also to enable any person skilled in the art to practice the invention, including making using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

**1.** Sensor for measuring amount of substance in blood the sensor comprising:
a base element (9) having a surface, and
at least one light emitting diode (10) emitting at pre-determined wavelength and a photodetector (11) which is sensitive to the pre-determined wavelength,
**characterized in that**
the at least one light emitting diode comprises at least one emissive layer (2; 4) the emissive layer having been printed onto the surface.

**2.** Sensor as claimed in claim 1, **characterized in that** the sensor comprises two light emitting diodes emitting at different wavelengths and having two emissive layers (2, 4) printed onto the surface.

**3.** Sensor as claimed in claim 1 or 2, **characterized in that** the sensor further comprises anode (1, 5) and cathode (3) layers.

**4.** Sensor as claimed in claim 3, **characterized in that** the layers (1 - 5) of the light emitting diodes have been printed on top of each other and that at least the layers printed on top of the emissive layer (2, 4) are made transparent.

**5.** Sensor as claimed in claim 3, **characterized in that** the emissive layers (2, 4) have been printed side by side with respect to each other and printed between the anode and cathode layers (1, 3, 5), and that at least the anode and cathode layers (1, 3) printed on top of the emissive layers (2, 4) are made transparent.

**6.** Sensor as claimed in claim 1, **characterized in that** a lens (19) has been arranged on top of the at least one light emitting diode.

**7.** Sensor as claimed in claim 1, **characterized in that** the sensor comprises a layer comprising printed conductive traces (6, 7, 8).

**8.** Sensor as claimed in claim 2, **characterized in that** the wavelengths of the at least two light emitting diodes (10) and the spectral response of the at least one photodetector (11) are within the ranges of 650 ... 670 nm and 880 ... 950 nm.

**9.** Sensor as claimed in claim 1, **characterized in that** sensor further comprises a printed photosensitive layer.

**10.** Method of making a sensor for measuring amount of substance in blood, **characterized in that** the method comprises the following steps:
providing a base element (9) having a surface, and
forming a structure having layers by printing the layers onto the surface, the layers comprising at least one emissive layer having a pre-determined wavelength, and anode and cathode layers.

**11.** Method as claimed in claim 10, **characterized in that** at least the layers printed on top of emissive layer (2, 4) are made as transparent layers.

**13.** Method as claimed in claim 10, **characterized in that** the base element (9) is a flexible or a rigid element.

**14.** Method as claimed in claim 10, **characterized in that** the emissive layers (2, 4) are printed side by side with respect to each other, and at least the anode and cathode layers (1, 3) printed on top of the emissive layers (2, 4) are made transparent.

**15.** Method as claimed in claim 10, **characterized in that** the method further comprises a step in which after the printing step each sensor is punched out from the base element (9).
